# FASCICULE DE BREVET EUROPEEN

(11) **EP 2 326 673 B1**
(45) Date de publication et mention de la délivrance du brevet: **08.08.2012**
(21) Numéro de dépôt: 09748410.9
(22) Date de dépôt: 18.09.2009
(51) Int. Cl.: C08F 2/38, C08F 212/08, C08F 220/06, C08F 220/18, C09D 125/14, C09J 125/14, A61K 8/81, A61Q 19/00

(54) **COPOLYMERES A GRADIENT DE COMPOSITION A MATRICE RIGIDE SOLUBLES ET/OU DISPERSIBLES DANS L'EAU ET DANS DES SOLVANTS ORGANIQUES**
COPOLYMERE MIT ZUSAMMENSETZUNGSGRADIENT UND STARRER MATRIX, DIE IN WASSER UND IN ORGANISCHEN LÖSUNGSMITTELN LÖSLICH UND/ODER DISPERGIERBAR SIND
RIGID-MATRIX COMPOSITION-GRADIENT COPOLYMERS WHICH ARE SOLUBLE AND/OR DISPERSIBLE IN WATER AND IN ORGANIC SOLVENTS

(30) Priorité: 19.09.2008 FR 0856309
(43) Date de publication de la demande: 01.06.2011
(73) Titulaire: Arkema France, 92700 Colombes (FR)
(72) Inventeur: MAGNET, Stéphanie, F-64370 Morlanne (FR); HEDIGER, Hélène, F-64150 Mourenx (FR); GUERRET, Olivier, F-69890 La Tour De Salvagny (FR)
(74) Mandataire: Albani, Dalila
(86) Numéro de dépôt international: PCT/FR2009/051760
(87) Numéro de publication internationale: WO 2010/031973

(56) Documents cités:
- WO-A-2007/140192
- WO-A-2008/079677
- US-A1- 2004 180 019
- US-A1- 2007 128 127

## Description

La présente invention a trait à des copolymères à gradient de composition à matrice rigide, solubles et/ou dispersibles dans l'eau et dans des solvants organiques obtenus par polymérisation radicalaire contrôlée, lesdits copolymères présentant un caractère amphiphile, à savoir qu'ils présentent à la fois une partie hydrophile et une partie hydrophobe.

La présente invention a trait également à un procédé de préparation de tels copolymères ainsi qu'à un procédé de mise en solution aqueuse de ces copolymères.

Ces copolymères trouvent leur application dans des domaines très variés, notamment des domaines nécessitant une mise en solution aqueuse de ce type de copolymères, tels que les domaines du traitement de surface, notamment les peintures, les adhésifs, les colles ou encore tels que le domaine cosmétique ainsi que dans la dispersion pigmentaire.

Ainsi, le domaine général de l'invention est celui des copolymères amphiphiles.

### ÉTAT DE LA TECHNIQUE ANTÉRIEURE

Les copolymères amphiphiles sont des copolymères comprenant dans leur structure au moins une partie hydrophile issue de la polymérisation de monomères porteurs d'au moins une fonction hydrophile et au moins une partie hydrophobe issue de la polymérisation de monomères porteurs d'au moins une fonction hydrophobe.

Ces copolymères peuvent être réalisés par différentes techniques de polymérisation telles que la polymérisation anionique, la polymérisation radicalaire classique ou encore la polymérisation radicalaire contrôlée.

Les copolymères amphiphiles issus de la polymérisation radicalaire classique sont généralement des copolymères statistiques, certains d'entre eux étant notamment connus sous le terme d'ASR (abréviation correspondant à l'expression anglaise « Alkali-Soluble Resin »). Ces copolymères sont formés à partir de monomère(s) hydrophobe(s) comme le styrène ou l'α-méthylstyrène et de monomère(s) hydrophile(s) comme l'acide acrylique ou l'acide méthacrylique. Parmi les ASR, on peut citer l'exemple des copolymères Joncryl de Johnson Polymer (résines styrène-acrylique), celui des Neocryl (copolymères styrène-acrylique) et des Haloflex (copolymères vinyl-acrylique).

L'inconvénient des copolymères statistiques réalisés par polymérisation radicalaire classique est qu'ils présentent des motifs répartis de façon non homogène entre les différentes chaînes de polymère. De cela peut découler le fait qu'une certaine partie des chaînes de polymère peut être très hydrophile, car comprenant une proportion élevée d'unités issues de la polymérisation de monomères hydrophiles tandis qu'une autre partie peut être très hydrophobe.

Une solution pour remédier aux problèmes inhérents à l'inhomogénéité de composition des chaînes de polymères est de réaliser la synthèse des polymères par polymérisation radicalaire contrôlée. Ce type de polymérisation permet ainsi de conduire à des copolymères dont les compositions chimiques des chaînes de polymère sont homogènes et proches d'une chaîne à l'autre.

Les copolymères amphiphiles classiquement issus de la polymérisation radicalaire contrôlée sont, pour la plupart des copolymères blocs, dont chaque bloc présente des propriétés particulières.

Cependant, les procédés de préparation de tels copolymères sont souvent longs et coûteux et font appel à une synthèse multi-étapes. En effet, la préparation de copolymères à blocs implique la succession d'au moins deux étapes de polymérisation, en vue de constituer au moins deux blocs, entre lesquelles s'insère une étape de dévolatilisation des monomères résiduels présents à la fin de la première étape.

Ainsi, les inventeurs se sont fixé comme objectif de proposer des copolymères à gradient de composition qui ne présentent pas les problèmes d'inhomogénéité de composition au sein d'une même chaîne ainsi que les problèmes inhérents à la synthèse des copolymères à blocs.

Des copolymères à gradient de composition, comprenant deux ou plusieurs monomères différents sont connus par exemple du document US 2007/0128127, qui enseigne qu'une composition comprenant de 2 à 25% en poids d'un monomère hydrophile, de 50 à 90% en poids d'un monomère de Tg inférieure ou égale à 20°C et de 5 à 25% en poids d'un monomère additionnel convient à une utilisation dans le traitement capillaire, en prévenant le blanchiment des cheveux lorsqu'elle est utilisée sous forme de formulation aqueuse additionnée de silicones. D'autres copolymères à gradient de composition sont décrits dans les documents WO 2008/079677, US 2004/180019 et WO 2007/140192.

La présente invention se propose de fournir de nouveaux copolymères à gradient de composition.

### EXPOSÉ DE L'INVENTION

Ainsi, l'invention a trait, selon un premier objet, à des copolymères à gradient de composition comprenant :
- des unités répétitives issues de la polymérisation d'au moins un premier monomère M₁ dont l'homopolymère correspondant présente une température de transition vitreuse Tg₁ inférieure à 20°C, lesdites unités répétitives représentant de 15 à 40% en masse, avantageusement de 20 à 35% en masse, par rapport à la masse totale du copolymère ;
- des unités répétitives issues de la polymérisation d'au moins un second monomère M₂ dont l'homopolymère correspondant présente une température de transition vitreuse Tg₂ supérieure à 20°C, lesdites unités répétitives représentant de 45% à 65% en masse, avantageusement de 50 à 62% en masse, par rapport à la masse totale du copolymère ;
- des unités répétitives hydrophiles issues de la polymérisation d'au moins un troisième monomère M₃, lesdites unités répétitives représentant de 10 à 25% en masse, avantageusement de 13 à 21 % en masse, par rapport à la masse totale du copolymère.

On précise que, par « copolymères à gradient de composition », on entend des copolymères, dans lesquels la composition locale en monomères change de manière continue le long d'une chaîne de polymère, cette composition étant fonction de la réactivité des monomères mis en présence. Les copolymères à gradient de composition sont à différencier des copolymères blocs, dans lesquels la composition locale change de manière discontinue le long de la chaîne et ils sont aussi à dissocier des copolymères statistiques, qui ne présentent pas non plus de variation continue de la composition.

Tg₁ est comprise entre -150°C et 20°C, de préférence entre -120°C et 15°C.

Les températures de transition vitreuse ont été mesurées par calorimétrie différentielle à balayage (DSC).

Les copolymères à gradient de composition de l'invention peuvent présenter une masse moléculaire moyenne en poids allant de 30 000 g/mol à 70 000 g/mol, de préférence de 40 000 g/mol à 60 000 g/mol et un indice de polymolécularité inférieur à 2.

Selon l'invention, le troisième monomère M₃, conférant le caractère hydrophile aux copolymères de l'invention, est choisi, avantageusement, parmi :
- les monomères éthyléniques comportant au moins un groupe carboxylique, tels que l'acide (méth)acrylique, l'acide itaconique, l'acide fumarique ;
- les monomères (méth)acrylates comportant au moins un groupe polyéthylèneglycol et/ou glycol éventuellement substitué sur leur fonction terminale par un groupe alkyle, phosphate, phosphonate ou sulfonate ;
- les monomères éthyléniques comportant au moins un groupe amide, tels que le (méth)acrylamide et leurs dérivés N-substitués ;
- les monomères (méth)acrylates d'aminoalkyle ;
- les monomères (méth)acrylamides d'aminoalkyle ;
- les monomères éthyléniques comprenant au moins un groupe anhydride d'acide, tels que l'anhydride maléique ou l'anhydride fumarique ;
- les monomères vinylamides, tels que la vinylpyrrolidone, la vinylacétamide ;
- les monomères vinylamines, tels que la vinylmorpholine, la vinylamine ;
- la vinylpyridine ; et
- les mélanges de ceux-ci.

En particulier, le troisième monomère M₃ peut être l'acide méthacrylique.

Le premier monomère M₁ est choisi, avantageusement parmi les acrylates d'alkyle dont l'homopolymère correspondant présente une température de transition vitreuse inférieure à 20°C, par exemple, les acrylates d'alkyle, linéaires ou ramifiés, en C₁-C₁₂, en particulier l'acrylate d'éthyle, les (méth)acrylates de polyéthylèneglycol et les monomères diéniques, tandis que le second monomère M₂ est choisi, avantageusement, parmi les monomères styréniques, en particulier le styrène, les monomères (méth)acrylates dont l'homopolymère correspondant présente une température de transition vitreuse supérieure à 20°C, tels que l'acrylate de norbomyle ou le méthacrylate de méthyle, le (méth)acrylonitrile.

Des copolymères à gradient de composition particuliers de l'invention sont des copolymères comprenant:
- des unités répétitives issues de la polymérisation d'au moins un premier monomère M₁, qui est l'acrylate d'éthyle, lesdites unités répétitives pouvant représenter 32% ou 23% en masse par rapport à la masse totale du copolymère ;
- des unités répétitives issues de la polymérisation d'au moins un second monomère M₂, qui est le styrène, lesdites unités répétitives pouvant représenter 53% ou 60% en masse par rapport à la masse totale du copolymère ; et
- des unités répétitives hydrophiles issues de la polymérisation d'au moins un troisième monomère M₃, qui est l'acide méthacrylique, lesdites unités répétitives pouvant représenter 15 ou 17% en masse par rapport à la masse totale du copolymère.

Les copolymères à gradient de composition de l'invention peuvent être préparés par un procédé comprenant les étapes suivantes :
a) une étape de polymérisation radicalaire contrôlée consistant à mettre en contact un mélange de monomères comprenant :
   * au moins un premier monomère M₁ dont l'homopolymère correspondant présente une température de transition vitreuse Tg₁ inférieure à 20°C, ledit premier monomère représentant de 15 à 40% en masse par rapport à la masse totale du mélange ;
   * au moins un second monomère M₂ dont l'homopolymère correspondant présente une température de transition vitreuse Tg₂ supérieure à 20°C, ledit second monomère représentant de 45% à 65% en masse par rapport à la masse totale du copolymère ; et
   * au moins un troisième monomère M₃ hydrophile ou comportant au moins un groupe apte à se transformer en une fonction hydrophile, ledit troisième monomère représentant de 10 à 25% en masse par rapport à la masse totale du copolymère,
      avec au moins un agent de contrôle et éventuellement un initiateur de polymérisation, si l'agent de contrôle n'est pas apte à initier une réaction de polymérisation ; et
b) éventuellement, une étape d'isolement dudit copolymère.

Plus précisément, l'étape de polymérisation a) se fait avantageusement sous une atmosphère de gaz inerte vis-à-vis des réactifs mis en jeu pour la polymérisation radicalaire contrôlée, tels que l'argon ou l'azote en présence éventuellement d'un solvant organique destiné à solubiliser les réactifs (monomères, agent de contrôle, initiateur éventuellement), ce solvant organique pouvant être un acétate d'alkyle, tel que l'acétate de butyle ou l'acétate d'éthyle. Il peut s'agir également d'un solvant aromatique, d'un solvant cétonique ou d'un solvant alcoolique.

L'étape de polymérisation s'effectue à une température choisie en fonction de la composition chimique du mélange de monomères, et en particulier de la constante cinétique de propagation des monomères et de l'affinité de ceux-ci pour l'agent de contrôle. Cette température peut être choisie dans une gamme allant de 10°C à 160°C, par exemple de 25°C à 130°C et de préférence de 50°C à 100°C.

Lors de l'étape de polymérisation, le mélange de monomères peut être ajouté en une fois ou sous forme continue sur la durée totale de l'étape de polymérisation.

L'étape de polymérisation est stoppée une fois la conversion souhaitée atteinte, sachant qu'il est préférable d'atteindre au moins 50% de conversion, de manière préférée, au moins 75% et encore plus préférentiellement au moins 90% de conversion.

A la fin de l'étape de polymérisation, il peut y avoir, le cas échéant, une étape d'élimination des éventuels monomères résiduels, soit par évaporation, soit par ajout d'une quantité d'initiateur classique de polymérisation, tel que les dérivés peroxydiques ou azoïques.

Enfin, le copolymère à gradient obtenu peut être isolé de son milieu de polymérisation lors de l'étape b).

Selon l'invention, l'agent de contrôle répond, avantageusement, à la formule (I) suivante : 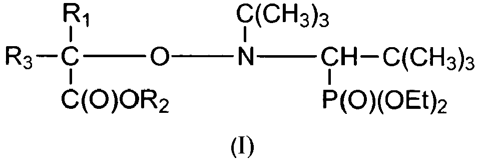 dans laquelle :
* R₁ et R₃, identiques ou différents, représentent un groupe alkyle, linéaire ou ramifié, ayant un nombre d'atomes de carbone allant de 1 à 3 ;
* R₂ représente un atome d'hydrogène, un groupe alkyle, linéaire ou ramifié, ayant un nombre d'atomes de carbone allant de 1 à 8, un groupe phényle, un métal alcalin tel que Li, Na, K, un ion ammonium tel que NH₄⁺, NHBu₃⁺; de préférence R₁ et R₃ étant CH₃ et R₂ étant H.

Un agent de contrôle particulier utilisable pour concevoir les copolymères à gradient de l'invention répond à la formule (II) suivante : 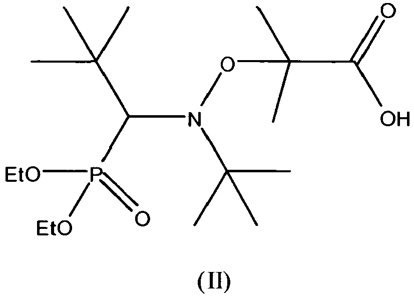 cet agent de contrôle étant désigné sous le nom de BlocBuilder.

L'agent de contrôle peut être également une alcoxyamine polyfonctionnelle répondant à la formule (III) suivante : 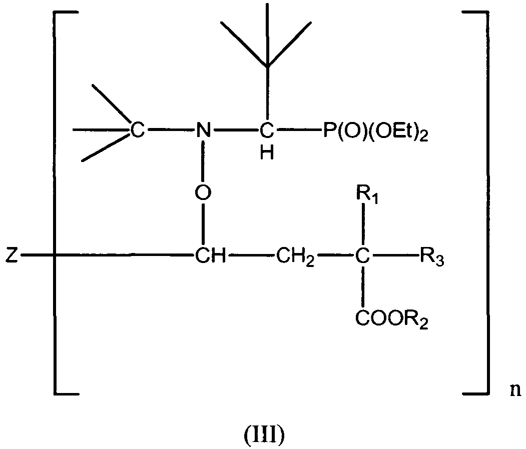 dans laquelle :
* R₁ et R₃, identiques ou différents, représentent un groupe alkyle, linéaire ou ramifié, ayant un nombre d'atomes de carbone allant de 1 à 3 ;
* R₂ représente un atome d'hydrogène, un groupe alkyle, linéaire ou ramifié, ayant un nombre d'atomes de carbone allant de 1 à 8, un groupe phényle, un métal alcalin tel que Li, Na, K, un ion ammonium tel que NH₄⁺, NHBu₃⁺ ; de préférence R₁ et R₃ étant CH₃ et R₂ étant H ;
* Z représente un groupe aryle ou un groupe de formule Z₁-[X-C(O)]ₙ, dans laquelle Z₁ représente une structure polyfonctionnelle provenant par exemple d'un composé du type polyol, X est un atome d'oxygène, un atome d'azote porteur d'un groupement carboné ou d'un atome d'hydrogène, ou un atome de soufre ; et
   - n est un nombre entier supérieur ou égal à 2.

On précise que l'abréviation Et correspond au groupe éthyle et que l'abréviation Bu correspond au groupe butyle, lequel peut exister sous différentes formes isomères (n-butyle, sec-butyle, tert-butyle).

Un exemple particulier d'agent de contrôle du type alcoxyamine polyfonctionnelle conforme à la définition générale donnée ci-dessus est l'alcoxyamine polyfonctionnelle répondant à la formule suivante :

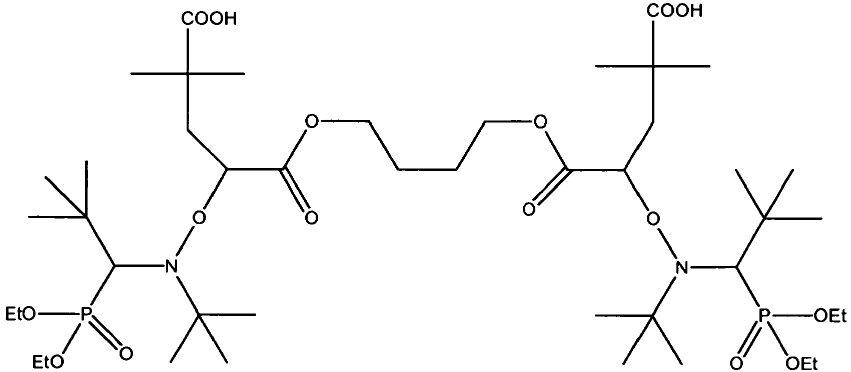

Le choix des monomères sera dicté par l'importance de situer les monomères hydrophiles à un endroit précis de la chaîne.

Ainsi, si l'on veut que les motifs hydrophiles soient dans le coeur de la chaîne polymère, on choisira préférablement un initiateur difonctionnel et un mélange de monomères tels que la réactivité des monomères hydrophiles est supérieure à celle des monomères hydrophobes. C'est le cas, par exemple, de l'acide méthacrylique par rapport aux monomères acrylates en général. Dans le cas où l'on veut des motifs hydrophiles à la périphérie, on choisira, par exemple, le couple acrylate/vinylpyrrolidone.

Les copolymères à gradient de l'invention sont hydrosolubles ou hydrodispersibles. Par copolymère hydrosoluble, on entend classiquement un copolymère pouvant former une solution limpide, lorsqu'il est en solution à 5% en masse dans l'eau, à une température de 25°C. Par copolymère hydrodispersible, on entend un copolymère apte à former, à une teneur de 5% en masse dans l'eau à 25°C, une suspension stable de fines particules, généralement sphériques. La taille moyenne des particules constituant ladite dispersion est inférieure à 1 µm et, plus généralement, varie entre 5 et 400 nm, de préférence de 10 à 250 nm, ces tailles moyennes de particules étant mesurées par diffusion de lumière.

C'est donc tout naturellement que les copolymères peuvent être mis en solution aqueuse.

Ainsi, l'invention a trait, selon un troisième objet, à un procédé de mise en solution aqueuse d'un copolymère à gradient tel que défini ci-dessus ou susceptible d'être obtenu par un procédé tel que défini ci-dessus comprenant les étapes suivantes :
a) dissoudre le copolymère dans une solution organique comprenant un solvant cétone ;
b) éventuellement, neutraliser ladite solution obtenue en a) par ajout d'une solution d'un acide ou d'une base ;
c) une étape d'ajout d'eau ou d'une solution aqueuse à la solution obtenue en a) ou b) ;
d) une étape d'évaporation de ladite solution organique.

Ainsi, la première étape consiste à dissoudre le copolymère dans une solution organique comprenant un solvant cétone, le copolymère étant classiquement dissous à raison d'une teneur allant de 20 à 90% en masse de la masse totale de la solution, de préférence de 20 à 50%, le solvant cétone pouvant être l'acétone ou la méthyléthylcétone.

Si besoin est, le procédé de l'invention comprend une étape de neutralisation destinée à neutraliser les fonctions acides et/ou basiques du copolymère. Plus précisément, dans le cas où le copolymère comprend des fonctions hydrophiles acides, l'étape de neutralisation peut consister à ajouter à la solution organique comprenant le copolymère une solution basique, de préférence d'au moins 1M, comprenant des ions hydroxonium OH⁻, des composés amines, des ions carbonates CO₃²⁻ ou hydrogénocarbonates HCO₃⁻. Dans le cas où le copolymère comprend des fonctions hydrophiles basiques du type amine, l'étape de neutralisation peut consister à ajouter à la solution organique comprenant le copolymère une solution acide, de préférence d'au moins 1M, telle qu'une solution d'acide chlorhydrique, d'acide bromhydrique, d'acide iodhydrique, d'acide acétique, d'acide propionique, d'acide sulfurique, d'acide phosphorique ou d'acide borohydrique.

Dans le cas où le copolymère comprend uniquement des fonctions hydrophiles telles que celles issues de monomères diméthylacrylamides ou N-vinylpyrrolidone, l'étape de neutralisation ne sera pas mise en oeuvre.

Après l'étape de mise en solution organique et l'éventuelle étape de neutralisation, le procédé comprend une étape d'ajout d'eau ou à tout le moins d'une solution aqueuse, avantageusement dans une proportion telle que le copolymère représente de 1 à 80% de la masse totale de la solution obtenue (solution organique + eau/solution aqueuse). La solution aqueuse, le cas échéant, peut être une solution comprenant de l'eau et un alcool dans des proportions pouvant aller de 99/1 et 50/50, l'alcool pouvant être l'éthanol et l'isopropanol.

Enfin, le procédé comprend une étape d'évaporation du solvant organique, jusqu'à obtention de la concentration en copolymère souhaitée. Cette étape d'évaporation peut consister en une étape de chauffage à une température suffisante pour l'évaporation du solvant organique.

L'invention a également trait à des compositions (ou solutions) aqueuses ou organiques comprenant au moins un copolymère tel que défini ci-dessus ou susceptible d'être obtenu par un procédé tel que défini ci-dessus, ces compositions aqueuses ou organiques ainsi que les copolymères de l'invention non mis en solution pouvant être utilisés dans le domaine des formulations de peintures, d'adhésifs, de colles destinées, notamment, à être appliquées sur des surfaces ayant peu d'affinité naturelle pour l'eau ou dans des formulations cosmétiques ou encore dans le domaine de la dispersion pigmentaire. Dans ces compositions, les copolymères sont, avantageusement, mis en solution dans l'eau ou dans un mélange eau-alcool, avantageusement, à des concentrations supérieures ou égales à 5% en masse.

L'invention va maintenant être décrite par rapport aux exemples suivants donnés à titre illustratif et non limitatif

### EXPOSÉ DÉTAILLÉ DE MODES DE RÉALISATION PARTICULIERS

Dans ces exemples, les masses molaires et leur distribution (indice de polymolécularité) ont été déterminées par chromatographie d'exclusion stérique, par calibrage universel utilisant des polystyrènes comme standard et les coefficients de Mark-Houwink du PMMA pour les copolymères.

La composition chimique des copolymères peut être déterminée par RMN du proton, spectrométrie UV, infrarouge.

### EXEMPLE 1

Dans un réacteur de 5 litres sont introduits à température ambiante 624 g d'acrylate d'éthyle, 729,6 g de styrène, 246,4 g d'acide méthacrylique, 400 g de méthyléthylcétone et 14,1 g de BlocBuilder (pureté=99%). Le milieu réactionnel est dégazé puis chauffé à 119°C. La température est maintenue pendant 200 minutes puis le milieu réactionnel est refroidi à température ambiante. La conversion obtenue est de 73%. 500 g de méthyléthylcétone sont alors additionnés et la solution polymérique est alors introduite dans un outil de dévolatilisation pour éliminer le solvant et les monomères résiduels. Le polymère est alors récupéré sous forme solide.

Le polymère présente les caractéristiques suivantes :
%Poly(acrylate d'éthyle) 32% massique
%Poly(styrène) 53% massique
%Poly(acide méthacrylique) 15% massique
Masse moléculaire en nombre (Mn) 20840 g/mol
Masse moléculaire au sommet du pic (Mp) 51280 g/mol
Masse moléculaire au poids (Mw) 46500 g/mol
Indice de polymolécularité (Ip) 1,9

### EXEMPLE 2

Dans un réacteur de 5 litres sont introduits à température ambiante 404 g d'acrylate d'éthyle, 928 g de styrène, 272 g d'acide méthacrylique, 400 g de méthyléthylcétone et 14,2 g de BlocBuilder (pureté=99%). Le milieu réactionnel est dégazé puis chauffé à 119°C. La température est maintenue pendant 255 minutes puis le milieu réactionnel est refroidi à température ambiante. La conversion obtenue est de 73%. 500 g de méthyléthylcétone sont alors additionnés et la solution polymérique est alors introduite dans un outil de dévolatilisation pour éliminer le solvant et les monomères résiduels. Le polymère est alors récupéré sous forme solide.

Le polymère présente les caractéristiques suivantes :
%Poly(acrylate d'éthyle) 23% massique
%Poly(styrène) 60% massique
%Poly(acide méthacrylique) 17% massique
Masse moléculaire en nombre (Mn) 22390 g/mol
Masse moléculaire au sommet du pic (Mp) 51680 g/mol
Masse moléculaire au poids (Mw) 47330 g/mol
Indice de polymolécularité (Ip) 1,9

## Revendications

1. Copolymère à gradient de composition comprenant :
- des unités répétitives issues de la polymérisation d'au moins un premier monomère M₁ dont l'homopolymère correspondant présente une température de transition vitreuse Tg₁ inférieure à 20°C, lesdites unités répétitives représentant de 15 à 40% en masse par rapport à la masse totale du copolymère ;
- des unités répétitives issues de la polymérisation d'au moins un second monomère M₂ dont l'homopolymère correspondant présente une température de transition vitreuse Tg₂ supérieure à 20°C, lesdites unités répétitives représentant de 45% à 65% en masse par rapport à la masse totale du copolymère ;
- des unités répétitives hydrophiles issues de la polymérisation d'au moins un troisième monomère M₃, lesdites unités répétitives représentant de 10 à 25% en masse par rapport à la masse totale du copolymère.

2. Copolymère à gradient selon la revendication 1, dans lequel lesdites unités répétitives issues de la polymérisation du premier monomère M₁ représentent de 20 à 35% en masse par rapport à la masse totale du copolymère.

3. Copolymère à gradient selon l'une des revendications 1 ou 2, dans lequel lesdites unités répétitives issues de la polymérisation du deuxième monomère M₂ représentent de 50 à 62% en masse par rapport à la masse totale du copolymère.

4. Copolymère à gradient selon l'une quelconque des revendications 1 à 3, dans lequel lesdites unités répétitives issues de la polymérisation du troisième monomère M₃ représentent de 13 à 21 % en masse par rapport à la masse totale du copolymère.

5. Copolymère à gradient selon l'une quelconque des revendications 1 à 4, dans lequel Tg₁ est compris entre -150°C et 20°C, de préférence entre -120°C et 15°C.

6. Copolymère à gradient selon l'une quelconque des revendications 1 à 5, présentant une masse moléculaire moyenne en poids allant de 30 000 g/mol à 70 000 g/mol, de préférence de 40 000 g/mol à 60 000 g/mol et un indice de polymolécularité inférieur à 2.

7. Copolymère à gradient selon l'une quelconque des revendications 1 à 6, dans lequel le troisième monomère M₃ est choisi parmi :
- les monomères éthyléniques comportant au moins un groupe carboxylique, tels que l'acide (méth)acrylique, l'acide itaconique, l'acide fumarique ;
- les monomères (méth)acrylates comportant au moins un groupe polyéthylèneglycol et/ou glycol éventuellement substitué sur leur fonction terminale par un groupe alkyle, phosphate, phosphonate ou sulfonate ;
- les monomères éthyléniques comprenant au moins un groupe amide, tels que le (méth)acrylamide et leurs dérivés N-substitués ;
- les monomères (méth)acrylates d'aminoalkyle ;
- les monomères (méth)acrylamides d'aminoalkyle ;
- les monomères éthyléniques comprenant au moins un groupe anhydride d'acide, tels que l'anhydride maléique ou l'anhydride fumarique ;
- les monomères vinylamides, tels que la vinylpyrrolidone, la vinylacétamide ;
- les monomères vinylamines, tels que la vinylmorpholine, la vinylamine ;
- la vinylpyridine ; et
- les mélanges de ceux-ci.

8. Copolymère à gradient selon l'une quelconque des revendications 1 à 7, dans lequel le premier monomère M₁ est choisi parmi les acrylates d'alkyle dont l'homopolymère correspondant présente une température de transition vitreuse inférieure à 20°C, les (méth)acrylates de polyéthylèneglycol et les monomères diéniques.

9. Copolymère à gradient selon l'une quelconque des revendications 1 à 8, dans lequel le second monomère M₂ est choisi parmi les monomères styréniques, les monomères (méth)acrylates dont l'homopolymère correspondant présente une température de transition vitreuse supérieure à 20°C, le (méth)acrylonitrile.

10. Copolymère à gradient selon l'une quelconque des revendications 1 à 9, qui est un copolymère comprenant :
- des unités répétitives issues de la polymérisation d'au moins un premier monomère M₁, qui est l'acrylate d'éthyle, lesdites unités répétitives pouvant représenter 32% ou 23% en masse par rapport à la masse totale du copolymère ;
- des unités répétitives issues de la polymérisation d'au moins un second monomère M₂, qui est le styrène, lesdites unités répétitives pouvant représenter 53% ou 60% en masse par rapport à la masse totale du copolymère ; et
- des unités répétitives hydrophiles issues de la polymérisation d'au moins un troisième monomère M₃, qui est l'acide méthacrylique, lesdites unités répétitives pouvant représenter 15 ou 17% en masse par rapport à la masse totale du copolymère.

11. Procédé de préparation d'un copolymère à gradient tel que défini selon l'une quelconque des revendications 1 à 10, comprenant les étapes suivantes :
a) une étape de polymérisation radicalaire contrôlée consistant à mettre en contact un mélange de monomères comprenant :
* au moins un premier monomère M₁ dont l'homopolymère correspondant présente une température de transition vitreuse Tg₁ inférieure à 20°C, ledit premier monomère représentant de 15 à 40% en masse par rapport à la masse totale du mélange ;
* au moins un second monomère M₂ dont l'homopolymère correspondant présente une température de transition vitreuse Tg₂ supérieure à 20°C, ledit second monomère représentant de 45% à 65% en masse par rapport à la masse totale du copolymère ; et
* au moins un troisième monomère M₃ hydrophile ou comportant au moins un groupe apte à se transformer en une fonction hydrophile, ledit troisième monomère représentant de 10 à 25% en masse par rapport à la masse totale du copolymère,
avec au moins un agent de contrôle et éventuellement un initiateur de polymérisation, si l'agent de contrôle n'est pas apte à initier une réaction de polymérisation ; et
b) éventuellement, une étape d'isolement dudit copolymère.

12. Procédé de préparation selon la revendication 11, dans lequel l'agent de contrôle est choisi parmi les composés de formules (I), (II) et (III) suivantes : 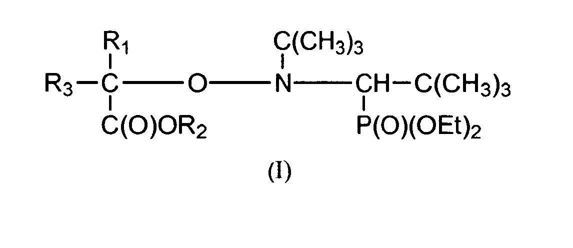 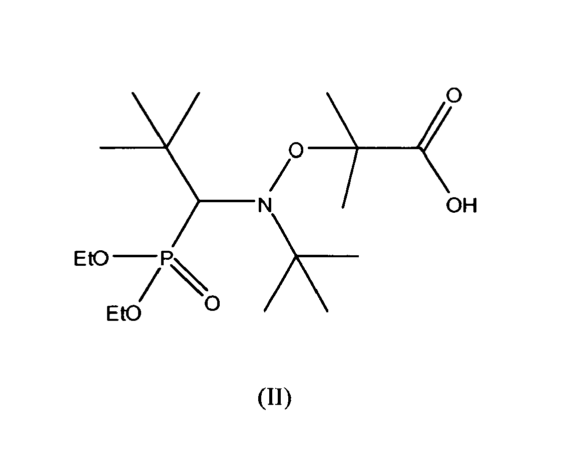 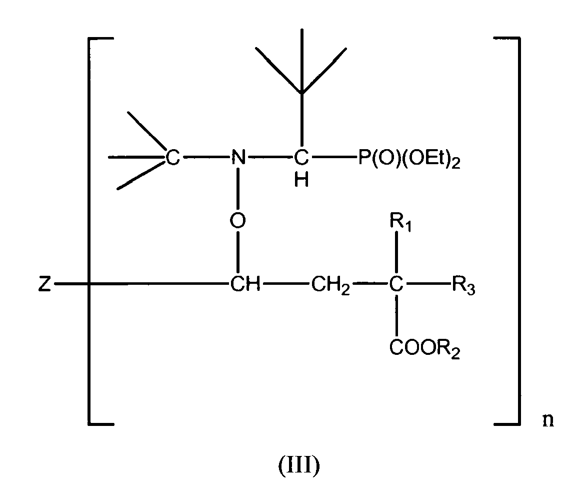 dans lesquelles :
* R₁ et R₃, identiques ou différents, représentent un groupe alkyle, linéaire ou ramifié, ayant un nombre d'atomes de carbone allant de 1 à 3 ;
* R₂ représente un atome d'hydrogène, un groupe alkyle, linéaire ou ramifié, ayant un nombre d'atomes de carbone allant de 1 à 8, un groupe phényle, un métal alcalin, un ion ammonium.
* Z représente un groupe aryle ou un groupe de formule Z₁-[X-C(O)]ₙ, dans laquelle Z₁ représente une structure polyfonctionnelle provenant par exemple d'un composé du type polyol, X est un atome d'oxygène, un atome d'azote porteur d'un groupement carboné ou d'un atome d'hydrogène, ou un atome de soufre ; et
- n est un nombre entier supérieur ou égal à 2.

13. Procédé de préparation selon l'une quelconque des revendications 11 à 12, dans lequel l'étape de polymérisation est réalisée à une température allant de 10 à 160°C.

14. Procédé de mise en solution aqueuse d'un copolymère à gradient tel que défini selon l'une quelconque des revendications 1 à 10 ou susceptible d'être obtenu par un procédé tel que défini selon l'une quelconque des revendications 11 à 13 comprenant les étapes suivantes :
a) dissoudre le copolymère dans une solution organique comprenant un solvant cétone ;
b) éventuellement, neutraliser ladite solution obtenue en a) par ajout d'une solution d'un acide ou d'une base ;
c) une étape d'ajout d'eau ou d'une solution aqueuse à la solution obtenue en a) ou b) ;
d) une étape d'évaporation de ladite solution organique.

15. Composition aqueuse ou organique comprenant au moins un copolymère, à gradient tel que défini selon l'une quelconque des revendications 1 à 10 ou susceptible d'être obtenu par un procédé tel que défini selon l'une quelconque des revendications 11 à 13, la concentration dudit copolymère à gradient étant égale ou supérieure à 5% en masse.

16. Utilisation d'au moins un copolymère à gradient tel que défini aux revendications 1 à 10 ou susceptible d'être obtenu par le procédé tel que défini aux revendications 11 à 13 ou d'au moins une composition aqueuse ou organique telle que définie à la revendication 15 dans des formulations de peintures, d'adhésifs, de colles, dans des formulations cosmétiques ou encore pour la dispersion pigmentaire.

## Claims

1. Composition-gradient polymer comprising:
- repeat units resulting from the polymerisation of at least one first monomer M₁, the corresponding homopolymer of which has a glass transition temperature Tg₁ of less than 20°C, said repeat units representing from 15% to 40% by mass relative to the total mass of the copolymer;
- repeat units resulting from the polymerization of at least a second monomer M₂, the corresponding homopolymer of which has a glass transition temperature Tg₂ of greater than 20°C, said repeat units representing from 45% to 65% by mass relative to the total mass of the copolymer;
- hydrophilic repeat units resulting from the polymerization of at least a third monomer M₃, wherein said repeat units represent from 10% to 25% by mass of the total mass of the copolymer.

2. Gradient copolymer according to claim 1, in which said repeat units resulting from the polymerisation of the first monomer M₁ represent from 20 to 35% by mass of the total mass of the copolymer.

3. Gradient copolymer according to one of claims 1 or 2, in which said repeat units resulting from the polymerisation of the second monomer M₂ represent from 50 to 62% by mass of the total mass of the copolymer.

4. Gradient copolymer according to any one of claims 1 to 3, in which said repeat units resulting from the polymerisation of the third monomer M₃ represent from 13 to 21% by mass of the total mass of the copolymer.

5. Gradient copolymer according to any one of claims 1 to 4, in which Tg₁ is between -150°C and 20°C, preferably between -120°C and 15°C.

6. Gradient copolymer according to any one of claims 1 to 5, presenting an average molecular mass by weight ranging from 30,000 g/mol to 70,000 g/mol, preferably from 40,000 g/mol to 60,000 g/mol and a polymolecularity index of less than 2.

7. Gradient copolymer according to any one of claims 1 to 6, in which the third monomer M₃ is chosen from among:
- the ethylenic monomers comprising at least one carboxylic group, such as (meth)acrylic acid, itaconic acid, fumaric acid;
- the (meth)acrylate monomers comprising at least one polyethyleneglycol and/or glycol optionally substituted on their terminal function by an alkyl, phosphate, phosphonate or sulfonate group;
- the ethylenic monomers comprising at least one amide group, such as (meth)acrylamide and their N-substituted derivatives;
- the aminoalkyl meth(acrylate) monomers;
- the aminoalkyl (meth)acrylamide monomers;
- the ethylenic monomers comprising at least one acid anhydride group, such as maleic anhydride or fumaric anhydride;
- the vinylamide monomers, such as vinylpyrrolidone, vinylacetamide;
- the vinylamine monomers, such as vinylmorpholine, vinylamine;
- vinylpyridine; and
- mixtures thereof.

8. Gradient copolymer according to any one of claims 1 to 7, in which the first monomer M₁ is chosen from among the alkyl acrylates, the corresponding homopolymer of which has a glass transition temperature of less than 20°C, the polyethyleneglycol (meth)acrylates and the dienic monomers.

9. Gradient copolymer according to any one of claims 1 to 8, in which the second monomer M₂ is chosen from among the styrenic monomers, the (meth)acrylate monomers the corresponding homopolymer of which has a glass transition temperature greater than 20°C, (meth)acrylonitrile.

10. Gradient copolymer according to any one of claims 1 to 9, which is a copolymer comprising:
- repeat units resulting from the polymerisation of at least one first monomer M₁, which is ethyl acrylate, said repeat units representing 32% or 23% by mass relative to the total mass of the copolymer;
- repeat units resulting from the polymerisation of at least one second monomer M₂, which is styrene, said repeat units representing 53% or 60% by mass relative to the total mass of the copolymer;
- hydrophilic repeat units resulting from the polymerisation of at least one third monomer M₃, which is methacrylic acid, said repeat units representing 15% or 17% by mass relative to the total mass of the copolymer.

11. Process for the preparation of a gradient copolymer as defined according to any one of claims 1 to 10, comprising the following steps:
a) a step of controlled radical polymerisation consisting of placing a mixture of monomers in contact, comprising:
* at least one first monomer M₁, the corresponding homopolymer of which has a glass transition temperature Tg₁ of less than 20°C, said monomer representing from 15% to 40% by mass relative to the total mass of the mixture;
* at least one second monomer M₂, the corresponding homopolymer of which has a glass transition temperature Tg₂ of greater than 20°C, said monomer representing from 45% to 65% by mass relative to the total mass of the copolymer; and
* at least one third monomer M₃, which is hydrophilic or comprises at least one group suitable for transforming to a hydrophilic function, said third monomer representing from 10% to 25% by mass of the total mass of the copolymer.
with at least one control agent and optionally a polymerisation initiator, if the control agent is not suitable for initiating a polymerisation reaction; and
b) optionally, a step of isolation of said copolymer.

12. Preparation process according to claim 11, in which the control agent is chosen from among the compounds of the following formulae (I), (II) and (III): 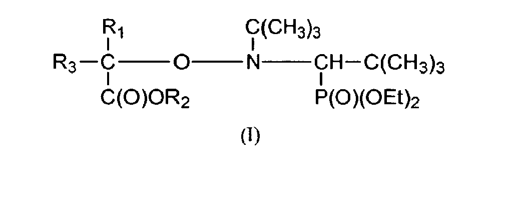 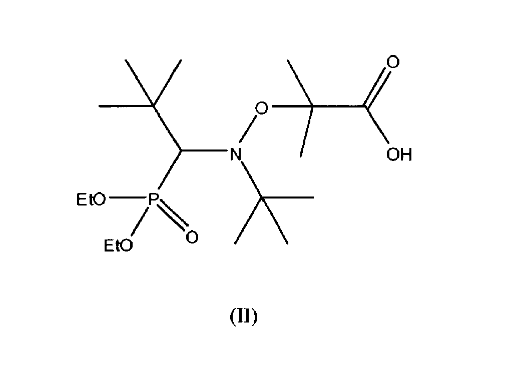 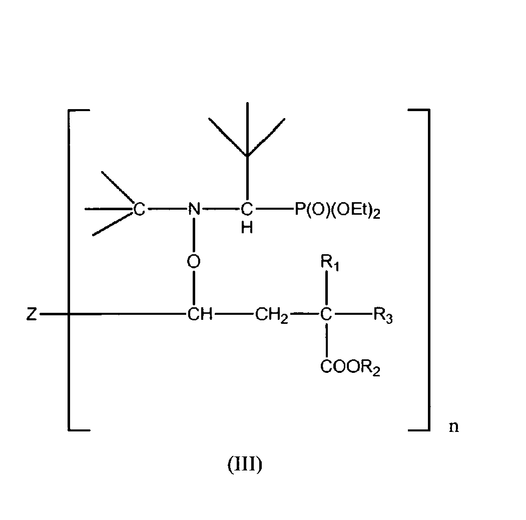 in which:
*R₁ and R₃, identical or different, represent a linear or branched alkyl group, with a number of carbon atoms ranging from 1 to 3;
* R₂ represents a hydrogen atom, a linear or branched alkyl group, with a number of carbon atoms ranging from 1 to 8, a phenyl group, an alkali metal, or an ammonium ion.
* Z represents an aryl group or a group of the formula Z₁-[X-C(O)]₂, in which Z₁ represents a polyfunctional structure resulting, for example, from a polyol-type compound, X is an oxygen atom, a nitrogen atom carrying a carbonated group or a hydrogen atom, or a sulphur atom; and
- n is a whole number greater than or equal to 2.

13. Preparation process according to any one of claims 11 to 12, in which the polymerisation step is carried out at a temperature from 10 to 160°C.

14. Process for aqueous dissolution of a gradient copolymer as defined according to any one of claims 1 to 10 or able to be obtained by a process such as defined according to any one of claims 11 to 13, comprising the following steps:
a) dissolving the copolymer in an organic solution comprising a ketone solvent;
b) optionally, neutralising said solution obtained in a) by adding a solution of an acid or a base;
c) a step of adding water or an aqueous solution to the solution obtained in a) or b);
d) a step of evaporation of said organic solution.

15. Aqueous or organic composition comprising at least one gradient copolymer as defined according to any one of claims 1 to 10 or able to be obtained by a process such as defined according to any one of claims 11 to 13, the concentration of said gradient copolymer being equal to or greater than 5% by mass.

16. Use of at least one gradient copolymer as defined in claims 1 to 10 or able to be obtained by the process as defined in claims 11 to 13 or of at least one aqueous or organic composition as defined in claim 15 in the formulation of paints, adhesives, glues, in cosmetic formulations or for pigment dispersion.

## Patentansprüche

1. Copolymer mit Zusammensetzungsgradienten, das aufweist:
- Wiederholungseinheiten, die aus der Polymerisation mindestens eines ersten Monomers M₁ stammen, dessen entsprechendes Homopolymer eine Glasübergangstemperatur T_{g1} unter 20 °C aufweist, wobei diese Wiederholungseinheiten 15 bis 40 Gew.-%, bezogen auf das Gesamtgewicht des Copolymers, ausmachen;
- Wiederholungseinheiten, die aus der Polymerisation mindestens eines zweiten Monomers M₂ stammen, dessen entsprechendes Homopolymer eine Glasübergangstemperatur T_{g2} über 20 °C aufweist, wobei diese Wiederholungseinheiten 45 bis 65 Gew.-%, bezogen auf das Gesamtgewicht des Copolymers, ausmachen;
- hydrophile Wiederholungseinheiten, die aus der Polymerisation mindestens eines dritten Monomers M₃ stammen, wobei diese Wiederholungseinheiten 10 bis 25 Gew.-%, bezogen auf das Gesamtgewicht des Copolymers, ausmachen.

2. Gradientencopolymer nach Anspruch 1, worin die Wiederholungseinheiten, die aus der Polymerisation des ersten Monomers M₁ stammen, 20 bis 35 Gew.-%, bezogen auf das Gesamtgewicht des Copolymers, ausmachen.

3. Gradientencopolymer nach einem der Ansprüche 1 oder 2, worin die Wiederholungseinheiten, die aus der Polymerisation des zweiten Monomers M₂ stammen, 50 bis 62 Gew.-%, bezogen auf das Gesamtgewicht des Copolymers, ausmachen.

4. Gradientencopolymer nach einem der Ansprüche 1 bis 3, worin die Wiederholungseinheiten, die aus der Polymerisation des dritten Monomers M₃ stammen, 13 bis 21 Gew.-%, bezogen auf das Gesamtgewicht des Copolymers, ausmachen.

5. Gradientencopolymer nach einem der Ansprüche 1 bis 4, wobei Tg₁ zwischen -150 und 20 °C und vorzugsweise zwischen -120 und 15 °C liegt.

6. Gradientencopolymer nach einem der Ansprüche 1 bis 5, das eine gewichtsmittlere Molmasse von 30000 bis 70000 g/mol, vorzugsweise 40000 bis 60000 g/mol und einen Polydispersitätsindex unter 2 aufweist.

7. Gradientencopolymer nach einem der Ansprüche 1 bis 6, worin das dritte Monomer M₃ ausgewählt ist unter:
- ethylenischen Monomeren, die mindestens eine Carboxygruppe aufweisen, wie (Meth)acrylsäure, Itaconsäure, Fumarsäure;
- (Meth)acrylatmonomeren, die mindestens eine Polyethylenglycol- und/oder Glycolgruppe aufweisen, die an ihrer endständigen Funktion gegebenenfalls mit Alkyl, Phosphat, Phosphonat oder Sulfat substituiert ist;
- ethylenischen Monomeren, die mindestens eine Amidgruppe aufweisen, wie (Meth)acrylamid und deren N-substituierten Derivaten;
- Aminoalkyl(meth)acrylatmonomeren;
- Aminoalkyl(meth)acrylamidmonomeren;
- ethylenischen Monomeren, die mindestens eine Säureanhydridgruppe aufweisen, wie Maleinsäureanhydrid oder Fumarsäureanhydrid;
- Vinylamidmonomeren, wie Vinylpyrrolidon, Vinylacetamid;
- Vinylaminmonomeren, wie Vinylmorpholin, Vinylamin;
- Vinylpyridin; und
- deren Gemischen.

8. Gradientencopolymer nach einem der Ansprüche 1 bis 7, worin das erste Monomer M₁ unter den Alkylacrylaten, deren entsprechendes Homopolymer eine Glasübergangstemperatur unter 20 °C aufweist, Polyethylenglycol(meth)acrylaten und Dienmonomeren ausgewählt ist.

9. Gradientencopolymer nach einem der Ansprüche 1 bis 8, worin das zweite Monomer M₂ unter den Styrolmonomeren, (Meth)acrylatmonomeren, deren entsprechendes Homopolymer eine Glasübergangstemperatur über 20 °C aufweist, (Meth)acrylnitril ausgewählt ist.

10. Gradientencopolymer nach einem der Ansprüche 1 bis 9, bei dem es sich um ein Copolymer handelt, das aufweist:
- Wiederholungseinheiten, die aus der Polymerisation mindestens eines ersten Monomers M₁ stammen, bei dem es sich um das Ethylacrylat handelt, wobei diese Wiederholungseinheiten 32 oder 23 Gew.-%, bezogen auf das Gesamtgewicht des Copolymers, ausmachen können;
- Wiederholungseinheiten, die aus der Polymerisation mindestens eines zweiten Monomers M₂ stammen, bei dem es sich um das Styrol handelt, wobei diese Wiederholungseinheiten 53 oder 60 Gew.-%, bezogen auf das Gesamtgewicht des Copolymers, ausmachen können; und
- hydrophile Wiederholungseinheiten, die aus der Polymerisation mindestens eines dritten Monomers M₃ stammen, bei dem es sich um die Methacrylsäure handelt, wobei diese Wiederholungseinheiten 15 oder 17 Gew.-%, bezogen auf das Gesamtgewicht des Copolymers, ausmachen können.

11. Verfahren zur Herstellung eines Gradientencopolymers nach einem der Ansprüche 1 bis 10, das die folgenden Schritte umfasst:
a) einen Schritt der kontrollierten radikalischen Polymerisation, der darin besteht, ein Gemisch von Monomeren, das umfasst:
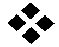 mindestens ein erstes Monomer M₁ dessen entsprechendes Homopolymer eine Glasübergangstemperatur Tg₁ unter 20 °C aufweist, wobei das erste Monomer 15 bis 40 Gew.-%, bezogen auf das Gesamtgewicht des Gemisches, ausmacht;
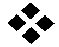 mindestens ein zweites Monomer M₂, dessen entsprechendes Homopolymer eine Glasübergangstemperatur Tg₂ über 20 °C aufweist, wobei das zweite Monomer 45 bis 65 Gew.-%, bezogen auf das Gesamtgewicht des Gemisches, ausmacht; und
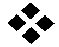 mindestens ein drittes Monomer M₃, das hyrophil ist oder das mindestens eine Gruppe aufweist, die befähigt ist, sich in eine hydrophile Gruppe umzuwandeln, wobei das dritte Monomer 10 bis 25 Gew.-%, bezogen auf das Gesamtgewicht des Gemisches, ausmacht,
mit mindestens einem Kontrollmittel und gegebenenfalls einem Polymerisationsinitiator in Kontakt zu bringen, wenn das Kontrollmittel nicht dazu in der Lage ist, die Polymerisation zu starten; und
b) gegebenenfalls einen Schritt der Isolierung des Copolymers.

12. Verfahren zur Herstellung nach Anspruch 11, worin das Kontrollmittel unter den Verbindungen der folgenden Formeln (I), (II) und (III) ausgewählt ist: 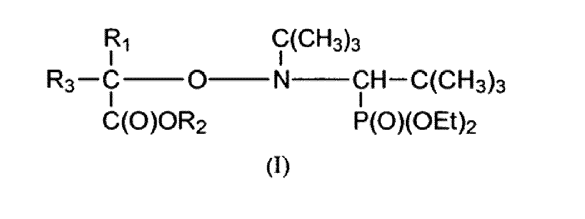 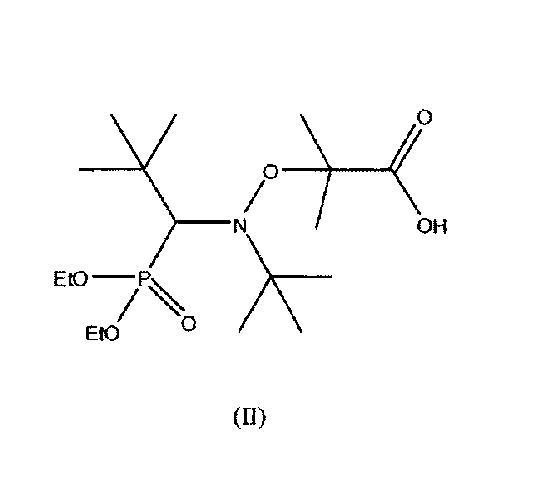 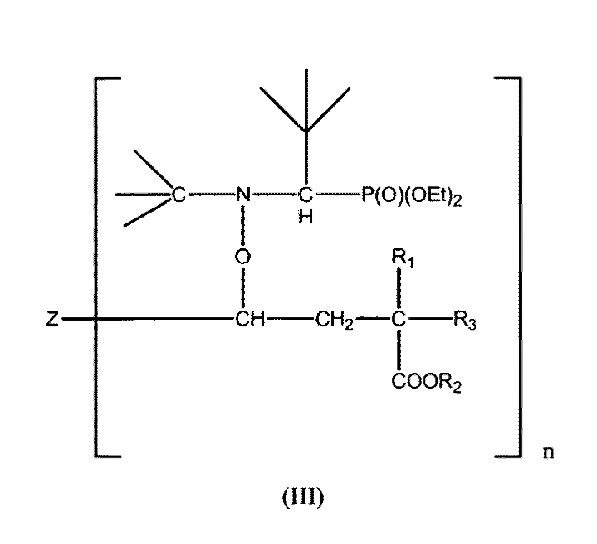 in den Formeln:
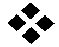 die Gruppen R₁ und R₃, die gleich oder verschieden sind, bedeuten eine geradkettige oder verzweigte Alkylgruppe mit einer Anzahl von Kohlenstoffatomen von 1 bis 3;
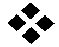 R₂ bedeutet ein Wasserstoffatom, eine geradkettige oder verzweigte Alkylgruppe mit einer Anzahl von Kohlenstoffatomen von 1 bis 8, eine Phenylgruppe, ein Alkalimetall, ein Ammoniumion;
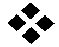 Z bedeutet eine Arylgruppe oder eine Gruppe der Formel Z₁-[X-C(O)]ₙ, worin Z₁ eine polyfunktionelle Struktur bedeutet, die beispielsweise von einer Verbindung vom Typ Polyol stammt, X ist ein Sauerstoffatom, ein Stickstoffatom, das eine Kohlenstoffgruppe oder ein Wasserstoffatom trägt, oder ein Schwefelatom; und
- n ist eine ganze Zahl größer oder gleich 2.

13. Verfahren zur Herstellung nach einem der Ansprüche 11 und 12, worin der Polymerisationsschritt bei einer Temperatur im Bereich von 10 bis 160 °C durchgeführt wird.

14. Verfahren zum Lösen eines wie in einem der Ansprüche 1 bis 10 definierten oder nach einem wie in einem der Ansprüche 11 bis 13 definierten Verfahren erhältlichen Gradientencopolymers in einer wässrigen Lösung, das die folgenden Schritte umfasst:
a) Lösen des Copolymers in einer organischen Lösung, die ein Ketonlösemittel enthält;
b) gegebenenfalls Neutralisieren der in a) erhaltenen Lösung durch Zusatz einer Säurelösung oder Basenlösung;
c) einen Schritt, in dem Wasser oder eine wässrige Lösung zu der in a) oder b) erhaltenen Lösung gegeben wird;
d) einen Schritt, in dem die organische Lösung verdampft wird.

15. Wässrige oder organische Zusammensetzung, die mindestens ein wie in einem der Ansprüche 1 bis 10 definiertes oder nach einem wie in einem der Ansprüche 11 bis 13 definierten Verfahren erhältliches Gradientencopolymer enthält, wobei die Konzentration des Gradientencopolymers größer oder gleich 5 Gew.-% ist.

16. Verwendung mindestens eines wie in einem der Ansprüche 1 bis 10 definierten oder nach einem wie in einem der Ansprüche 11 bis 13 definierten Verfahren erhältlichen Gradientencopolymers oder mindestens einer wie in Anspruch 15 definierten wässrigen oder organischen Zusammensetzung in Formulierungen für Lacke, Klebstoffe, Kleber, in kosmetischen Formulierungen oder auch für Pigmentdispersionen.
